# EUROPEAN PATENT APPLICATION

(11) **EP 2 818 560 A1**
(43) Date of publication of application: **31.12.2014**
(21) Application number: 13174406.2
(22) Date of filing: 28.06.2013
(51) Int. Cl.: C12Q 1/68, G01N 33/53

(54) **Method for analysis of a nucleic acid-binding substance in a test sample**

(71) Applicant: aevotis GmbH, 14473 Potsdam (DE)
(72) Inventor: La Cognata, Ursula, 14163 Berlin (DE); Landschütze, Volker, 12203 Berlin (DE); Lippold, Felix, 14471 Potsdam (DE); Gabor, Peggy, 15827 Blankenfelde (DE)
(74) Representative: Beyer, Andreas

(57) **Abstract**

Method for analysis of a nucleic acid-binding substance in a test sample, comprising
a) formation of an analysis mixture, comprising
a test sample to by analyzed for presence of nucleic acid-binding substance, and a nucleic acid
wherein in the analysis mixture nucleic acid-binding substance binds to the nucleic acid,
b) determination of the presence or the amount of free nucleic acid in the analysis mixture, wherein free nucleic acid is nucleic acid that is not bound to nucleic acid-binding substance,
c) determination of the presence or the amount of nucleic acid-binding substance in the analysis mixture.

## Description

The present invention relates to a method for analysis of a nucleic acid-binding substance in a test sample.

Polyethylene imine (PEI) is the polymerization product of ethylene imine. PEI is a nucleic acid-binding polymer whose amino groups can be protonated in aqueous environment. A typical, but non limiting molecular weight is about 30 kDa.

PEI is a very "sticky" compound which interacts with negatively charged molecules, like DNA, and precipitates them. In biotechnological processes PEI can be used for purification of enzymes produced by genetically modified microorganisms. In purification of cell extracts PEI is used as a flocculent to decrease the turbidity and to facilitate further processing steps. In addition, it binds the plasmid DNA which is from a recombinant production organism.

The drawback of PEI is a very strong toxicity and it has to be removed. When enzymes are produced for foodstuff applications, it has to be ensured that the level of PEI in cell extracts is below a given threshold, typically below 0.1 ppm.

Thus, there is a need for a method for detection of PEI in an enzyme/protein solution to verify the level is below a given threshold, as for example 0.1 ppm, which ensures food grade material.

There are other nucleic acid binding substances than cationic polymers, such as PEI. Excess of such substances remains in samples after binding, precipitation and/or flocculation of nucleic acids, for example in biological or analytical samples. Generally, there is a need for effective analysis for nucleic acid-binding substances, particularly in liquid samples.

According to a general idea of the invention, nucleic acid is added to a sample, preferably in known amount, in order to detect the presence and/or amount of a nucleic acid binding substance in the sample.

The invention particularly provides with a method for analysis of a nucleic acid-binding substance according to claim 1. Special embodiments are subject matter of dependent method claims.

The invention provides with a method for analysis of the presence, amount and/or concentration of a nucleic acid-binding substance in a test sample, comprising
a) formation of an analysis mixture, comprising
   - a test sample, preferably liquid test sample, to be analyzed for presence, amount and/or concentration of a nucleic acid-binding substance, and
   - nucleic acid
      wherein in the analysis mixture nucleic acid-binding substance, if present in the test sample, binds to the nucleic acid,
b) determination of the presence, amount and/or concentration of free nucleic acid in the analysis mixture, wherein free nucleic acid is nucleic acid that is not bound to nucleic acid-binding substance,
c) determination of the presence, amount and/or concentration of nucleic acid-binding substance in the analysis mixture and/or in the test sample.

Presence, amount and/or concentration of nucleic acid-binding substance in the test sample can be determined after determination of the presence, amount and/or concentration of nucleic acid-binding substance in the analysis mixture. It is also possible, to determine the presence, amount and/or concentration of nucleic acid-binding substance in the test sample directly.

The term "nucleic acid-binding substance" means a nucleic acid-binding chemical substance and is also just abbreviated as "substance" in the following description of the invention. The term "nucleic acid-binding substance" comprises any substance that binds to nucleic acid, as for example ribonucleic acid (RNA) or deoxyribonucleic acid (DNA). The term "nucleic acid-binding substance" preferably means a substance that coagulates, precipitates and/or flocculates nucleic acid in/from solution. Thus, the nucleic acid-binding substance can be a nucleic acid-coagulating agent (also designated as coagulant), a nucleic acid-flocculating agent (also designated as flocculent), and/or a nucleic acid-precipitating agent (also designated as precipitant).

An example of a nucleic acid-binding substance is a nucleic acid-binding chemical compound.Specific examples of a chemical compound are inorganic substances, as inorganic salts, oxides or compounds. Examples of inorganic substances are Aluminiumsulphate, Polyaluminium chloride ferrous sulphate, Polyaluminium chloride ferric sulphate, Ferrous chloride, Ferric chloride, Ferric chloride - sulphate, Aluminium chloride, Lime (CaO), Calcium chloride, Magnesium chloride, Na₂Al₂O_{4.} Said specific compounds also have the function as coagulation agents.

Further examples of inorganic compounds are silica compounds with flocculating properties, such as amorphous, silica, activated silica, diatomite (Celite), Perlite. Other inorganic compounds are colloidal clays, such as bentonite, or metallic hydroxides with polymeric structure, such as Al hydroxides and Fe-hydroxides. These specific compounds also have the function as flocculating agents.

A still more specific example of a "nucleic acid-binding substance" is a nucleic acid-binding oligomer or polymer, preferably an oligomer or a polymer that precipitates or flocculates nucleic acid in/from solution. The term "nucleic acid-binding oligomer or polymer" is also abbreviated as "polymer" in the following description of the invention. I.e. the term "polymer" is also intended to comprise oligomers, which are short chain polymers.

Examples of polymers are natural polymers with nucleic acid-binding properties, such as starch derivatives, polysaccharides, particularly guar gum, and alginates.

Further examples of polymers are synthetic polymers with nucleic acid-binding properties, such as polyacrylamides, copolymer of acrylamide with cationic monomer, copolymer of acrylamide with acrylic acid, polyethylenimine, polyamidamine, polyamine, polyethylenoxide. All these polymers have the function as flocculation agents, i.e. they are nucleic acid-flocculating polymers.

A specific example of a nucleic acid-binding polymer, and of a nucleic acid-flocculating polymer, is a cationic polymer. The term "cationic polymer" comprises polymers with permanent positive charge as well as polymers that can be positively charged in aqueous environment, preferably acidic to neutral aqueous environment, due to protonation. Thus, a further specific example of a nucleic acid-binding polymer is polymer with properties of a base, also designated as a "basic polymer". Basic properties can originate from groups or functional moieties that can be protonated. In a special embodiment the cationic polymer is a nitrogen containing polymer. The cationic polymer may, for example, comprise amino groups. A specific example of a cationic polymer is polyethylene imine (PEI). Specific examples of cationic polymers are polyethylene imine (PEI), copolymer of acrylamide with a cationic monomer.

In the method of the invention the binding properties of cationic polymer, as for example PEI, to negatively charged nucleic acids, as for example ribonucleic acid (RNA) or deoxyribonucleic acid (DNA), single strand or double strand, can be used to determine the presence, absence or amount of cationic polymer in a sample.

Presenceof nucleic acid-binding substance can be detected, for example, when no free nucleic acid can be detected. The term "determination of the presence, amount and/or concentration of substance" also includes the special case that no nucleic acid-binding substance can be detected, i.e. the determination of absence. Absence of nucleic acid-binding substance may be detected when free nucleic acid is present and can be detected and when all nucleic acid in the analysis mixture is found as free nucleic acid.

The term "nucleic acid", or "nucleic acid molecule", comprises polynucleotides, particularly ribonucleic acid (RNA) and deoxyribonucleic acid (DNA), which can be single stranded or double stranded.The nucleic acid can be obtained for instance from natural sources or may be produced synthetically or by recombinant techniques.

The presence of free nucleic acid may also indicate that nucleic acid-binding substance is present below a certain threshold, as exemplified in the appended examples. In a specific embodiment, step c) means determination that nucleic acid-binding substance is below a threshold or a critical value.

Preferably, a known amount of nucleic acid is added to the analysis mixture. Said known amount of nucleic acid is completely or partially transformed to bound nucleic acid, if nucleic acid-binding substance is present and binds to all of the nucleic acid or to a part thereof.

When nucleic acid-binding substance is present in a sample it depends on the amount of nucleic acid-binding substance whether free nucleic acid is still present in the analysis mixture. If the amount of substance is high enough in relation to nucleic acid, all free nucleic acid is bound by substance, and transferred to so-called "bound nucleic acid", and no free nucleic acid can be detected in step b). This leads to the conclusion in step c) that substance is present in the analysis mixture, and in the test sample that is part of the analysis mixture. In case that all nucleic acid is found as free nucleic acid in step b) the conclusion can be drawn that no substance is present in the analysis mixture, and in the test sample that is part of the analysis mixture. Between the two extreme cases there is the case that a part of a known amount of total nucleic acid in the analysis mixture is determined in step b) as free nucleic acid. From the amount of free nucleic acid the amount of bound nucleic acid can be determined and the amount of substance can be estimated.

It is to be understood determination of the presence or absence of free nucleic acid is related to the sensitivity of the method that is used for analysis. Thus, the result "absence of free nucleic acid" does not necessarily mean that absolutely no free nucleic acid is present, but it means that no free nucleic acid is detectable any more by the respective method.

In the method of the invention, detection of free nucleic acid can be carried out with electrophoresis, by staining the DNA e.g. with Gelred or ethidiumbromide and visualize it under an UV imager, but in principle all other techniques for nucleic acid determination can be employed. Electrophoresis, or UV spectroscopy, Fluorimetric Quantitation or Hybridization-Based Techniques other technique, is preferably combined with known nucleic-acid marking techniques in order to make nucleic acids detectable. Useful markers are for example Gelred and ethidium bromide. By using markers, the presence, absence or amount of nucleic acid can be determined. Calibration samples with varying amounts of free nucleic acid may be employed in order to determine the amount of free nucleic acid in the analysis mixture.

In one embodiment of a method of the invention the analysis mixture comprises components for amplifying the nucleic acid, particularly a DNA, by a Polymerase Chain Reaction, and the analysis mixture is subjected to conditions of a Polymerase Chain Reaction. Said components for amplifying the nucleic acid by a Polymerase Chain Reaction (hereinafter PCR) are known components necessary for a PCR besides the nucleic acid that is to be amplified. Components of a PCR, as for example primer(s), DNA polymerase, particularly Taq-Polymerase,cation(s), such as Mg²⁺, Mn²⁺ and/or K⁺, buffer, and deoxynucleosidetriphosphates (dNTP), are known to a skilled person. Suitable primers are employed that are complementary to the ends of each of the sense and anti-sense strand of a chosen DNA target. So, when the analysis mixture comprises components for amplifying the nucleic acid by a Polymerase Chain Reaction, the analysis mixture preferably comprises primer(s), polymerase, cation(s), buffer and deoxy nucleoside triphosphates.

Also conditions of a Polymerase Chain Reaction, i.e. thermal cycling, are commonly known. Steps of the PCR are a) Denaturation, b) Primer Annealing and c) Elongation. So, the term "subjected to conditions of a Polymerase Chain Reaction" preferably means that the analysis mixture is subjected to at least one denaturation step, at least one primer annealing step and at least one elongation step. Steps a)-c) of the PCR are preferably done 10-50 times. Preferably 10-50 cycles are done, wherein each cycle comprises steps a)-c). The steps are done with an analysis mixture that comprises the components for amplifying the nucleic acid by a Polymerase Chain Reaction. Before the first cycle is done, a so-called Initialization step may be done. After the last cycle, a so-called final elongation step may be done. Temperature and time parameters of the mentioned steps may be chosen as follows, wherein these conditions are only an example and not to be construed to limit the invention, because PCR as such is a standard technique, well known to the skilled person: Initialization: 85-98°C, 10 - 900 seconds;
Denaturation (step a): 85-98°C, 10 - 600 seconds;
Primer Annealing (step b): 45-70°C, 10- 600 seconds;
Elongation (step c): 65-80°C, 10 - 600 seconds;
Final elongation: 65-80°C, 10 - 900 seconds
The temperature of elongation is chosen higher than the temperature of primer annealing. Typically the annealing temperature is about 2-7 °C below the melting temperature of the primers used. The temperature at the elongation step depends on the DNA polymerase used. The elongation time depends both on the DNA polymerase used and on the length of the DNA fragment to be amplified.

Using PCR makes the method very sensitive for detection of small amounts or concentrations of nucleic acid-binding substance, particularly nucleic acid-binding polymer, preferably below 0.1 ppm. The analysis mixture may only contain a relatively small amount or concentration of nucleic acid. If there is a high enough amount or concentration of nucleic acid-binding substance in the analysis mixture for binding the whole amount of nucleic acid, the PCR reaction is inhibited. So, the term "subjected to conditions of a Polymerase Chain Reaction" includes the case that no PCR reaction happens and that consequently, no amplification products of a PCR reaction can be detected in step b). In this regard, the expression "determination of the presence, amount and/or concentration of free nucleic acid" means determination of the presence, amount and/or concentration of PCR amplification products, or, on the other hand, determination of the absence of PCR amplification products.

Since the amount of nucleic acid necessary as starting material for a PCR reaction is relatively small, only a small amount of nucleic acid binding substance is necessary to bind all nucleic acid and to inhibit the PCR reaction. An exemplary, but not limiting amount of nucleis acid, particularly DNA, in a PCR mixture is in the range of 0.5 pg (0.0005 ng) to 50 ng (=50000 pg).

As shown more detailed in the example, the nucleic acid binding character of the substance, such as the "sticky" character of the specific compound PEI, can be used to calculate the amount of nucleic acid binding substance via a PCR loaded with a fixed amount of nucleic acid.Without wishing to be bound by theory, it is believed that bound nucleic acid cannot be amplified by a PCR reaction any more. In result, no PCR product, i.e. no amplified free nucleic acid, can be detected in step b) and the conclusion can be drawn (step c) that nucleic acid-binding substance is present in analysis mixture. If free nucleic acid remains in the analysis mixture, it can be amplified and further free nucleic acid be formed by PCR. The amplification product can easily be detected due to its amount formed in the amplification process.

In a further embodiment, the method further comprises the following steps:
i. Formation of calibration mixtures, comprising predetermined ratios, predetermined amounts and/or predetermined concentrations, of nucleic acid-binding substance and nucleic acid,
ii. determination of the presence, amount and/or concentration of free nucleic acid in the calibration mixtures,
iii. determination of a detection limit for the nucleic acid-binding substance.

The presence, amount and/or concentration of nucleic acid-binding substance in the analysis mixture can be determined by use of the detection limit, as explained hereinafter.

The expression "predetermined ratio/amount/concentration" means that the ratio/amount/concentration is known in advance.

In this embodiment, the method of the invention comprises calibration steps. Steps i, ii, and iii may be done before or after steps a)-c) that were explained above.

In a preferred embodiment, a known amount of nucleic acid is given in each calibration mixture, preferably the same amount in each calibration mixture, and different amounts of nucleic acid-binding substance are given in each calibration mixture. Increasing amounts of nucleic acid-binding substance lead to increased binding to nucleic acid until all nucleic acid is bound to nucleic acid-binding substance.

The detection limit, which can also be designated as a boundary value, is preferably
- a ratio of nucleic acid-binding substance and nucleic acid, and/or
- an amount of nucleic acid-binding substance related to a specific amount or concentration of nucleic acid, and/or
- a concentration of nucleic acid-binding substance related to a specific amount or concentration of nucleic acid
wherein free nucleic acid still can still be detected, or wherein free nucleic acid cannot be detected anymore in a calibration mixture.

The amount or concentration of nucleic acid in the analysis mixture(s) and the amount or concentration of nucleic acid in the calibration mixture(s) may be identical.

In a more specific embodiment the steps are done as follows:
In the formation of calibration mixtures a series of calibration mixtures with increasing ratio of nucleic acid-binding substance to nucleic acid is formed, and a last calibration mixture in the series wherein the presence of free nucleic acid is determined and/or a first calibration mixture in the series wherein no presence of free nucleic acid is determined, is/are identified,
and the detection limit(s) is/are determined.

As an alternative, a series of calibration mixtures with decreasing ratio of nucleic acid-binding substance to nucleic acid is formed, and a last calibration mixture in the series wherein no presence of free nucleic acid is determined and/or afirst calibration mixture in the series wherein the presence of free nucleic acid is determined, is/are identified, and the detection limit(s) is/are determined.

The detection limit is determined by use of the identified calibration mixture. Preferably, the amount or concentration of nucleic acid-binding substance in the identified calibration mixture is taken as detection limit.

Said ratio of nucleic acid-binding substance to nucleic acid in calibration mixtures may for example be expressed as a molar ratio of nucleic acid-binding substance to nucleic acid, or as a mass-ratio of nucleic acid-binding substance to nucleic acid, or as a ratio of molar amount of nucleic acid-binding substance to mass of nucleic acid, or as a ratio of mass of nucleic acid-binding substance to molar amount of nucleic acid.

Generally, amounts may for example be expressed in molar amounts or in mass amounts. Concentrations may for example be expressed as molar concentrations, particularly molar amount/volume, or mass concentrations, particularly mass/volume.

In one specific embodiment, step iii comprises the determination of the minimum ratio of nucleic acid-binding substance to nucleic acid that inhibits the detection of free nucleic acid in a calibration mixture, or determination of the minimum amount of nucleic acid-binding substance that inhibits the detection of free nucleic acid in a calibration mixture, or determination of the minimum concentration of nucleic acid-binding substance that inhibits the detection of free nucleic acid in a calibration mixture. Said minimum ratio/amount/concentration can be taken as detection limit. For example, calibration mixtures with no detectable free nucleic acid are identified and, preferably, calibration mixtures with still detectable free nucleic acid are also identified. From calibration mixtures with no detectable free nucleic acid the mixture with the smallest ratio of nucleic acid-binding substance to nucleic acid, or the smallest amount or concentration of nucleic acid-binding substance, is identified. Thereby the minimum ratio of nucleic acid-binding substance to nucleic acid, or the minimum amount or concentration of nucleic acid-binding substance, that inhibits the detection of free nucleic acid in a calibration mixture is identified. The more different ratios of liquid test sample to nucleic acid are examined, the more precisely the detection limit may be determined. A minimum amount or concentration of nucleic acid-binding substance can be calculated from a known ratio of nucleic acid-binding substance to nucleic acid in the respective calibration sample. Said minimum ratio, amount or concentration that inhibits formation of free nucleic acid can be taken as detection limit, as stated above. If in an analysis mixture free nucleic acid is detected, it can be concluded that the amount of nucleic acid-binding substance is below a certain value: If the amounts of nucleic acid in the calibration mixture and in the analysis mixture are the same, it can be concluded that the amount of nucleic acid-binding substance in the analysis mixture is below the detection limit. This is because if nucleic acid-binding substance was present in the amount or concentration of this detection limit, no free nucleic acid could be detected. If the amounts of nucleic acid in the calibration mixture and in the analysis mixture are not the same, it can be concluded that the amount of nucleic acid-binding substance in the analysis mixture is below a certain value, calculated by use of the detection limit and the amounts of nucleic acid in the calibration mixture and in the analysis mixture.

In another specific embodiment, step iii comprises the determination of the maximum ratio of nucleic acid-binding substance to nucleic acid, or determination of the maximum amount of nucleic acid-binding substance, or determination of the maximum concentration of nucleic acid-binding substance, wherein free nucleic acid in a calibration mixture can still be detected. For example, calibration mixtures with still detectable free nucleic acid are identified and, optionally, calibration mixtures with no detectable free nucleic acid are also identified in order to estimate the boundary between the last calibration mixture that comprises detectable free nucleic acid and the first calibration mixture that does not comprise free nucleic acid. From the calibration mixtures with still detectable free nucleic acid the mixture with the highest ratio of nucleic acid-binding substance to nucleic acid, or the highest amount or concentration of nucleic acid-binding substance, is identified. Thereby the maximum ratio of nucleic acid-binding substance to nucleic acid, or the maximum amount or concentration of nucleic acid-binding substance, that still does not inhibit the detection of free nucleic acid in a calibration mixture is identified. Said maximum amount can be calculated from the known ratio of nucleic acid-binding substance to nucleic acid in the respective sample. Said maximum ratio, amount or concentration can be taken as detection limit. The more different ratios of liquid test sample to nucleic acid are examined, the more precisely the detection limit may be determined. If in an analysis mixture free nucleic acid is detected, the detection limit can be used to estimate or to calculate the maximum amount or concentration of nucleic acid-binding substance in an analysis mixture and/or in a test sample, as explained in the appended examples. If in an analysis mixture free nucleic acid is detected, it can be concluded that nucleic acid-binding substance is lower or equal than a certain value: If the amounts of nucleic acid in the calibration mixture and in the analysis mixture are the same, it can be concluded that the amount of nucleic acid-binding substance in the analysis mixture is lower or equal than the detection limit. This is because if nucleic acid-binding substance was present in the amount or concentration of the detection limit defined in this way, free nucleic acid could still be detected. If the amounts of nucleic acid in the calibration mixture and in the analysis mixture are not the same, it can be concluded that the amount of nucleic acid-binding substance in the analysis mixture is below a certain value, calculated by use of the detection limit and the amounts of nucleic acid in the calibration mixture and in the analysis mixture.

The detection limit can be chosen as needed for a specific application. For example if a maximum permissible value for nucleic acid-binding substance in a test sample or in an analysis mixture, for example an enzyme solution, is known, the detection limit may be chosen accordingly. The detection limit can be varied by variation of the amount of nucleic acid in the calibration mixture. A desired detection limit can be set by choosing an appropriate amount of nucleic acid. In other words: the detection limit depends on the amount or concentration of nucleic acid used. This is further explained in the appended examples.

In case that a test sample is diluted in order to form the analysis mixture by a certain factor, said factor, also called "dilution factor" may be used to estimate or to calculate the amount or concentration of nucleic acid-binding substance in the test sample. The amount or concentration may be an amount-range (such as mass amount or molar amount) or a concentration range.

The maximum amount or concentration of nucleic acid-binding substance in an analysis mixture or in a test sample can be compared with a given maximum acceptable amount or concentration in the analysis mixture or in the test sample and it can be estimated whether the amount or concentration of nucleic acid-binding substance is equal or below said maximum acceptable amount or concentration. A minor error in this statement may result from the chosen ratios of nucleic acid-binding substance to nucleic acid, or amounts of nucleic acid-binding substance, or concentrations of nucleic acid-binding substance in the calibration mixtures. A maximum acceptable amount or concentration is a special case of a threshold-value.

In a special embodiment, the calibration mixtures comprise components for amplifying the nucleic acid by a Polymerase Chain Reaction (PCR), and the calibration mixtures are subjected to conditions of a Polymerase Chain Reaction. Application of PCR in the calibration mixtures is preferably similar, and has the same advantages and effects, as explained above with respect to the analysis mixtures. Usual components of a PCR were already mentioned above. When the calibration mixture comprises components for amplifying the nucleic acid by a Polymerase Chain Reaction, the calibration mixture preferably comprises primer(s), polymerase, cation(s), buffer and deoxynucleosidetriphosphates.

Also in connection with calibration mixtures, the term "subjected to conditions of a Polymerase Chain Reaction" preferably means that the calibration mixtures are subjected to at least one denaturation step, at least one primer annealing step and at least one elongation step. Further details, preferred embodiments and preferred conditions of these steps were already indicated above and can be applied here in similar manner. Preferably, PCR technique is used in both in calibration mixtures and in analysis mixtures, more preferably with the same given amount of nucleic acid in both cases. In connection with PCR, the expression "inhibition of the detection of free nucleic acid in a calibration mixture" means inhibition of PCR amplification of a given amount of nucleic acid that is added to a calibration mixture, and consequently inhibition of detection of amplification products.

In one embodiment, the method of the invention comprises:
- formation of a series of analysis mixtures with different ratios of test sample to nucleic acid,
- determination of an amount, a concentration, particularly an amount-range or a concentration-range, of nucleic acid-binding substance in the analysis mixture and/or in the test sample, by use of the detection limit for the nucleic acid-binding substance.

Also in this connection, the term "determination of an amount, a concentration, particularly an amount-range or a concentration-range" includes the special case that no nucleic acid-binding substance can be detected, i.e. the determination of absence.

The series comprises two or more analysis mixtures. In this embodiment, even more precise information about the amount (range) or a concentration (range) of nucleic acid-binding substance in the analysis mixture and/or in the test sample can be obtained. The detection limit gives information about the amount, a concentration, an amount-range or a concentration-range of nucleic acid-binding substance in the analysis mixture and also in the test sample. In this embodiment, it can not only be determined only whether the amount or concentration of nucleic acid-binding substance is lower equal, or higher than a detection limit, or lower, equal or higher than a value derived from the detection limit, but an even more precise amount (range) or concentration (range) can be determined, as shown in examples.

Formation of a series of analysis mixtures with different ratios of liquid test sample to nucleic acid may mean that a series of increasing or decreasing ratio of liquid test sample to nucleic acid is formed. Several analysis mixtures with different ratios of liquid test sample to nucleic acid are analyzed for presence of free nucleic acid.

Preferably, all analysis mixtures comprise the same, given amount of nucleic acid. The term "ratio" in this connection may be a ratio of volume or amount of test sample to amount of nucleic acid, wherein an amount can be expressed in amount of substance, preferably moles, or in mass. In the analysis mixtures, the total volume may be kept constant. A constant volume may be used, for example, when the analysis mixtures comprise components for amplifying the nucleic acid by a Polymerase Chain Reaction, and when the analysis mixtures are subjected to conditions of a Polymerase Chain Reaction.

In case that a test sample is diluted by a certain factor in order to form the analysis mixture, said factor, also called "dilution factor" may be used to estimate or to calculate the amount or concentration of nucleic acid-binding substance in the test sample, as explained in the appended examples. A general dilution factor of ≥1 may be applied wherein a dilution factor of exactly 1 means no dilution and a factor of 2 means that the test sample a diluted 1:1, for example that x microliter liquid test sample are diluted with the same volume of x microliter of another liquid, wherein said another liquid may comprise nucleic acid and, in a specific embodiment, components for amplification by PCR. A preferred range of the dilution factor is 1 to 10, more preferably 1 to 5.

When analyzing several analysis mixtures with different ratios of liquid test sample to nucleic acid for presence of free nucleic acid, different cases may occur, such as
A. all of the analysis mixtures contain free nucleic acid,
B. a part of the several analysis mixtures contains free nucleic acid,
C. none of the analysis mixtures contains free nucleic acid.

Case A leads to the conclusion that in all analysis mixtures the amount of nucleic acid-binding substance is so low that not all nucleic acid is bound. If for example the detection limit is defined as the maximum amount or concentration of nucleic acid-binding substance, that still does not inhibit the detection of free nucleic acid, and if the amount of nucleic acid in the calibration mixtures and in the analysis mixtures are the same, case A leads to the conclusion that the amount or concentration of nucleic acid-binding substance in all analysis mixtures is below the detection limit or equal to the detection limit. If the amounts of nucleic acid in the calibration mixtures and in the analysis mixtures are not the same, the amount or concentration of nucleic acid-binding substance in all analysis mixtures can be determined by using the detection limit and the amounts of nucleic acid. By using a dilution factor, if applicable, for example of ≥1, the amount or concentration of nucleic acid-binding substance in the test samples can be determined. It can then be checked whether the level of nucleic acid-binding substancein a test sample is below a given threshold.

Case C leads to the conclusion that in all analysis mixtures the amount of nucleic acid-binding substance is so high that all nucleic acid is bound. If for example the detection limit is defined as the maximum amount or concentration of nucleic acid-binding substance, that still does not inhibit the detection of free nucleic acid, and if the amount of nucleic acid in the calibration mixtures and in the analysis mixtures are the same, case C leads to the conclusion that the amount or concentration of nucleic acid-binding substance in the analysis mixtures is higher than the detection limit. It can then be checked whether the level of nucleic acid-binding substance in a test sample is below a given threshold.

Case B is in between these two extreme cases, i.e. it comprises case A and case B. Two specific embodiments are as follows:
- All analysis mixtures with no detectable free nucleic acid may be identified. Then the smallest ratio of liquid test sample to nucleic acid that leads to such negative result can be determined. Then, a detection limit that reflects the minimum ratio of nucleic acid-binding substance to nucleic acid that inhibits the detection of free nucleic acid in a calibration mixture, or that reflects the minimum amount of nucleic acid-binding substance that inhibits the detection of free nucleic acid in a calibration mixture, or that reflects the minimum concentration of nucleic acid-binding substance that inhibits the detection of free nucleic acid in a calibration mixture, can be used to determine the amount or concentration of nucleic acid-binding substance in the analysis mixtures and/or the text sample, and/or
- all analysis mixtures with still detectable free nucleic acid may be identified. Then the highest ratio of liquid test sample to nucleic acid that leads to such positive result can be determined. Then, a detection limit that reflects the maximum ratio of nucleic acid-binding substance to nucleic acid, or that reflects the maximum amount or maximum concentration of nucleic acid-binding substance, that still does not inhibit the detection of free nucleic acid in a calibration mixture can be used to determine the amount or concentration of nucleic acid-binding substance in the analysis mixtures and/or the text sample.

The liquid sample to be analyzed may be a cell extract. The extract may result from genetically modified microorganisms that express a desired protein. Microorganisms are usually be disrupted to give a cell extract. Nucleic acid-binding substance, such as cationic polymer, particularly PEI, is added to the cell extract for purification. After the purification step, the presence or amount of remaining nucleic acid-binding substance can be determined according to the method of the invention. It can then be decided whether nucleic acid-binding substance is already below a desired threshold or whether it has to be removed in a further step.

The method of the invention can be performed with any nucleic acid binding substance, particularly any inorganic substance, any organic substance, more specifically with any polymer, that binds to nucleic acids, particularly to RNA, single strand DNA or double strand DNA. In a special embodiment apolymer is a nitrogen containing polymer. The polymer may, for example, comprise amino groups. A specific example of a cationic polymer is polyethylene imine (PEI).

In a further aspect, the invention relates to a kit, comprising the necessary components for performing the method of the invention. The kit preferably does not comprise the means and equipment for determination of the presence, amount and/or concentration of free nucleic acid. The kit particularly comprises nucleic acid-binding substance and nucleic acid, wherein the kit is adapted to form calibration mixtures, comprising predetermined ratios, predetermined amounts and/or predetermined concentrations, of nucleic acid-binding substance and nucleic acid.

The invention in a further aspect is directed to a set of calibration mixtures, comprising predetermined ratios, predetermined amounts and/or predetermined concentrations, of nucleic acid-binding substance and nucleic acid, wherein the calibration mixtures are adapted to be analyzed on the presence, amount and/or concentration of free nucleic acid in the calibration mixtures.

The invention is further described by means of the following non limiting working examples.

Brief description of figures:
- Fig. 1: result of calibration, different ratios of PEI to DNA;
- Fig. 2: result of calibration, different ratios of PEI to PCR-mix with given amount of DNA;
- Fig. 3: results with different ratios of test samples to PCR-mix with given amount of DNA;

### Example 1 - Polyethylenimine (PEI) detection in alternansucrase enzyme solution

• The specification of the enzyme solution is that PEI levels have to be ≤ 0.1 ppm.
• Mix of 20ng pUC19 DNA with different amounts of PEI for calibration curve (detection limit); final vol. 50µl
• Incubation time PEI (Sigma) 1 h at 4°C
• Run 1 % TBE agarose gel

Fig: 1 shows the results of calibration (only lanes 4-11 are shown)
1 20ng pUC19 + PEI 0.02ppm
2 20ng pUC19 + PEI 0.04ppm
3 20ng pUC19 + PEI 0.06ppm
4 20ng pUC19 + PEI 0.08ppm
5 20ng pUC19 + PEI 0.10ppm
6 20ng pUC19 + PEI 0.12ppm
7 20ng pUC19 + PEI 0.14ppm
8 20ng pUC19 + PEI 0.16ppm
9 20ng pUC19 + PEI 0.18ppm
10 20ng pUC19 + PEI 0.20ppm
11 20ng pUC19 without PEI (control)

Detection limit: Concentration of last lane with visible band; here lane 6 = 0.12 ppm final concentration.

Use 50 µl test solution and mixed with 20ng pUC19 DNA. If DNA band is still visible, PEI level is ≤ 0.1 ppm (PEI is ≤ 0.12 ppm but only the first decimal place is shown because one decimal place is in this example sufficient to fulfill the specification).

### Calculation:

Dilution Factor = 11 (dilution factor) multiplied with 0.12 (detection limit) = 0.12. Thus the PEI level is ≤ 0.1 ppm.

### Example 2- Polyethylenimine (PEI) detection by Polymerase Chain Reaction (PCR) for alternansucrase enzyme solution

### Abbreviations

- DNAse: Desoxyribonuclease
- DSP: Downstream process
- PEI: Polyethylenimine
- EDTA: Ethylenediamine tetraacetic acid
- MgCl₂: Magnesiumchloride
- dNTPs: deoxyribonucleotide triphosphates: dATP, dCTP, dGTP and dTTP mix
- PCR: Polymerase chain reaction
- RNAse: Ribonuclease
- RO: Reverse osmosis
- TBE: Tris boric acid EDTA
- rpm: Rounds per minute
- UV: Ultraviolet

### 1. Purpose

Example 1 describes the determination of PEI in an alternansucrase enzyme/protein solution to verify the level is ≤ 0.1 ppm to ensure food grade material. The enzyme alternansucrase is produced via fermentation in a recombinant *Escherichia coli* expression system. During the DSP to partially purify the enzyme solution after fermentation, the PEI is used as a flocculent to decrease the turbidity and to facilitate further processing steps like filtration. In addition, it binds the plasmid DNA from the recombinant production organism which has to be removed as well.

### 2. Principle of method

The PEI used is a polymer with a molecular weight of about 25kDa with branched molecules. Each monomer carries positive charges. It is a very "sticky" compound which interacts with negatively charged molecules, like DNA, and precipitates them. This characteristic is used to calculate the amount of PEI in solution via PCR. The PCR is loaded with a fixed amount of pUC19 DNA and primers, which are small DNA fragments, too. 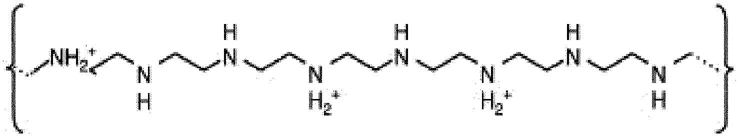 Chemical structure of PEI

### 3. Reagents

| **Name** | **Supplier** | **Cat. No.** |
|---|---|---|
| Agarose | Biozym | 840004 |
| DNAse (2000U/mg) | Roche Diagnostics | 10104159001 |
| Gel-Red | Biotium | 41003 |
| MgCl₂(50mM) | Invitrogen | 10342020 |
| dATP (part of dNTP) | Invitrogen | 5508-2 |
| dCTP(part of dNTP) | Invitrogen | 5508-3 |
| dGTP(part of dNTP) | Invitrogen | 5508-4 |
| dTTP (part of dNTP) | Invitrogen | 5508-5 |
| PCR buffer (10x) | Invitrogen | 10342020 |
| PEI | Sigma | 408727 |
| Primers (0.2µmol) | TIB MolBiol | - |
| pUC19 plasmid DNA | New England Biolabs | N3041S |
| RNAse (2000U/mg) | Roche Diagnostics | 10109134001 |
| RO water | Millipore | - |
| Size marker (100bp ladder) | Invitrogen | 15628-019 |
| TaqDNA polymerase | Invitrogen | 10342020 |
| TBE | Invitrogen | 15581-028 |

### 4. Working solutions

- TBE (1x): Dilute TBE (10x) 1:10 with RO water and use fresh as running buffer and for gel preparation.
- Agarose-gel: Prepare 1.5% (w/v) in TBE (1x), cook in a microwave until the agarose powder is melted , cool down to about 60°C, add Gel Red 1:10 000 (v/v) and use the mixture for your gel preparation.
- DNAse(0.3mg/ml): 3mg is weighed and dissolved in 1 ml RO water. Dilute1:10 in RO water and store refrigerated 2-8°C for up to 1 month.
- RNAse (0.3mg/ml): 3mg is weighed and dissolved in 1 ml RO water. Dilute1:10 in RO water and store refrigerated 2-8°C for up to 1 month.
- dNTPs (10mM each): 10µl of each TP (100mM) is added to 60µl RO water to make a 100µl solution with a final concentration of 10mM each. Store at -20°C for up to 12 months from the date of preparation.
- Primers (5µM): Prepare a 5 µM working solution from the primers (0.2µmol) with RO water and store at -20°C.
- pUC19 (1ng/ml): Prepare a 1µg/µl Stock by dilution 1:1 mio in two 1:1000 steps with RO water and store at -20°C.
- 20% PEI: 20g is weighed and dissolved in 100 ml of RO water (20% PEI stock; store refrigerated 2-8°C for maximum 1 year).

For the appropriate PEI amounts to add to the PCR, further serial dilutions are necessary (A-D). All dilutions are prepared with RO water and are freshly made before use.
A. 1:20 of stock (final concentration 10mg/ml PEI)
B. 1:100 of A (final concentration 0.1mg/ml = 100µg/ml PEI)
C. 1:100 of B (final concentration 1µg/ml)
D. 1:10 of C (final concentration 0.1µg/ml)

### 5. Specimen

The sample from Alternansucrase production is stored prior to analysis at -20 °C.

### 6. Procedure

One test enzyme solution is tested as follows: 1 sample preparation is divided into 4 PCRs-using volumes of 10µl, 15µl, 20µl and 25µl that are separately analyzed on one gel. Therefore, 4 results will be available for the evaluation of the sample. A twelve-well-gel can be used for the analysis of two samples (eight wells), 1 negative control, 1 positive control, 1 lane for the 100bp ladder.

### 6.1 Enzyme solution treatment

Take 250µl of the enzyme test solution and centrifuge it for 5 minutes at 10,000 rpm. Take 200µl of the supernatant and add 2µl of DNAse and RNAse working solutions each. The enzyme solution is pre-treated with DNAse/RNAseto eliminate all remaining DNA (plasmid and genomic). This is done for 5 minutes at room temperature. After incubation the digestion is stopped for 15 minutes at 95°C and centrifuged (5 minutes at 10,000 rpm). Digestion and centrifugation do not influence PEI concentrations (data available). Use the supernatant for PCR reactions.

### 6.2 PCR conditions

The assays are run in a final volume of 50µl.

In each sample 570nmol(1pg) DNA of plasmid pUC19 (containing ß-lactamase gene; C. Yanisch-Perron et al. [1985]: Improved M13 phage cloning vectors and host strains: nucleotide sequences of the M13mp18 and pUC19 vectors. In: Gene. Bd. 33, S. 103-119)is added as control.

As can be seen in comparison to example 1, wherein no PCR is used, the chosen amount of DNA is much lower, 1 pg in comparison to 20 ng.

Primers amplifying full-length(878bp) ß-lactamase gene are used and to the final mixture contains0.2mM dNTPs, 3mM MgCl₂,2.5U Taq Polymerase from Invitrogen and buffer.
- forward primer: gAgTATgAgTATTCAACATTTCCg (SEQ ID NO: 1)
- reverse primer: CTTggTCTgACAgTTACCAATgC (SEQ ID NO: 2)

Set up PCR reactions as follows:

| | |
|---|---|
| pUC19 DNA (1 pg) | 1µl* |
| Alternansucrase test solution** | different amounts up to 25µl |
| PEI | only for calibration curve - amounts see 6.3.2. |
| 10X PCR Buffer | 5 µl |
| dNTP mix (10 mM each) | 1 µl |
| forward Primer (5µM) | 2.4µl |
| reverse Primer (5µM) | 2.4 µl |
| add with RO water to 49.5µl | |
| Taq Polymerase (5 U/µl) | 0.5µl |

| | |
|---|---|
| * not for negative control (W); PCR reaction without pUC19 DNA and without test solution ** not for calibration curve | |

In an Eppendorf "Mastercycler Gradient" the assays run with 3 min preheating of 94°C and 30 cycles with 40 sec denaturation, an annealing temperature of 55°C for 40 sec and elongation for 1 min at 72°C.

Load thermocycler and run the following program:

| Step | Temperature | Time | Cycle |
|---|---|---|---|
| HeatSoak | 94°C | 3 minutes | 1x |
| Denaturation | 94°C | 40 seconds | 30x |
| Annealing | 55 °C | 40 seconds | 30x |
| Extension | 72 °C | 1 minute | 30x |
| Final Extension | 72 °C | 10 minutes | 1x |

Analyze 10 µl on a 1X TBE, 1.5 % agarose gel.

### 6.3 Gel electrophoresis

Use a 1.5% TBEagarose gel. Calibration and test samples are run on the same gel. Load 10µl of the 50µl PCR reaction. Run at RT with 100V for about 2h. Use the 100bp ladder (10µl) from Invitrogenas size marker.

### 6.3.1 Definition of visible band

Only bands of a size of 878 bp are relevant, which is between band eight and nine of the size marker when counted from the bottom. Use GelQuant.Net (BioChemLabSolutions.com) or equivalent program for band evaluation. Set negative control as background. All negative values and values below 0.01 are no visible bands.

### 6.3.2 Definition of visible band

For calibration a concentration curve of different PEI concentrations added to 570nmol (1pg) of pUC19 DNA is prepared. This is done fresh for each determination.

As negative control (W) no pUC19 DNA is added. Spike PCR load with appropriate volume (µl) from PEI-Stock E for lanes 1-6. Spike PCR load with appropriate volume (µl) from PEI-Stock D for lanes 7-9. Lane 10 is the positive control without any PEI.

| **lane** | **1** | **2** | **3** | **4** | **5** | **6** | **7** | **8** | **9** |
|---|---|---|---|---|---|---|---|---|---|
| Stock C(µl) | - | - | - | - | - | - | 2 | 4 | 5 |
| Stock D (µl) | 5 | 6 | 7 | 8 | 9 | 10 | - | - | - |
| Final PEI concentration (ppm) | 0.010 | 0.012 | 0.014 | 0.016 | 0.018 | 0.02 | 0.04 | 0.08 | 0.1 |

### Example for calculation:

5µl from 0.1 µg/ml stock D = 0.0005µg in 50µl volume = 0.00001 µg/µl = 0.01µg/ml= 0.01ppm

Run the PCR according to section 6.2. Run the gel according to section 6.3.
- The table above shows the final PEI concentrations (ppm)in lanes 1-9.
- Lanes 1-10 are spiked with 570nmol pUC19 plasmid DNA

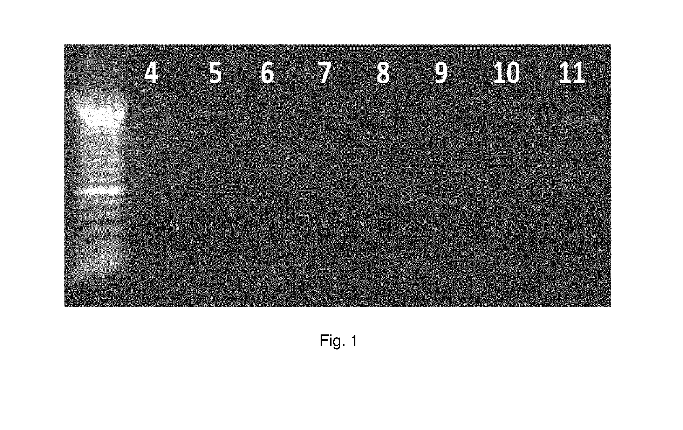

Fig.1 shows the result of the calibration gel.
- W: water control
- 1.: 0.010 ppm final PEI concentration
- 2.: 0.012 ppm
- 3.: 0.014 ppm
- 4.: 0.016 ppm
- 5.: 0.018 ppm
- 6.: 0.02 ppm
- 7.: 0.04 ppm
- 8.: 0.08 ppm
- 9.: 0.1 ppm
- 10.: Control pUC19

The Picture was taken under UV light. In the literature, pattern below 100bp as in lanes 1-6 are usually described as primer dimers, which are precipitated by PEI in lanes 7-9.

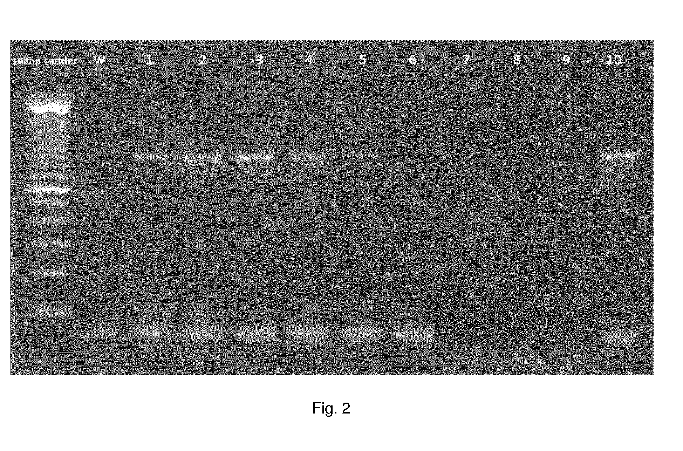

Detection limit: Concentration of last lane with visible band in Fig. 2; here lane 5= 0.018ppm final concentration.

As can be seen in comparison to example 1, wherein no PCR is used, and wherein the chosen amount of DNA is much lower, the detection limit is much lower: 0.018 ppm in comparison to 0.12 ppm. It can be seen by comparison of examples 1 and 2 that the detection limit is dependent on the chosen amount of nucleic acid.

### 6.3.3 Alternansucrase enzyme solution testing - calculation

System suitability:
a. Correct 100 bp ladder distributions on the gel (according to supplier instructions).
b. In the negative control, no band must be visible at 878 bp size, which is between band eight and nine of the size marker counted from the bottom up.
c. In the positive control, an 878 bp size has to be visible.
d. The sample evaluation can only be performed if these two conditions are fulfilled.

Sample preparation:
Different amounts of sample (10µl, 15µl, 20µl and 25µl) complemented with water should be tested. Run the PCR according to section 6.2. and load the gel according to section 6.3. The same amount of DNA is used as in the PCR done with the calibration mixtures (570 nmol, 1 pg).

Calculation:
Define the highest amount of enzyme test solution that has a visible band in this test.
Calculate the dilution factor by dividing the PCR volume by the defined amount:
   Dilution factor = PCR volume [µl] / enzyme volume [µl]
   with PCR volume = 50 µl, enzyme volume= highest amount of enzyme solution with visible DNA band on gel

Multiply the detection limit from the calibration curve with the calculated dilution factor:
Maximum PEI amount= Detection limit * dilution factor

The detection limit is 0.018, as determined in item 6.3.2.

The result is the maximum amount of PEI in the enzyme test solution.

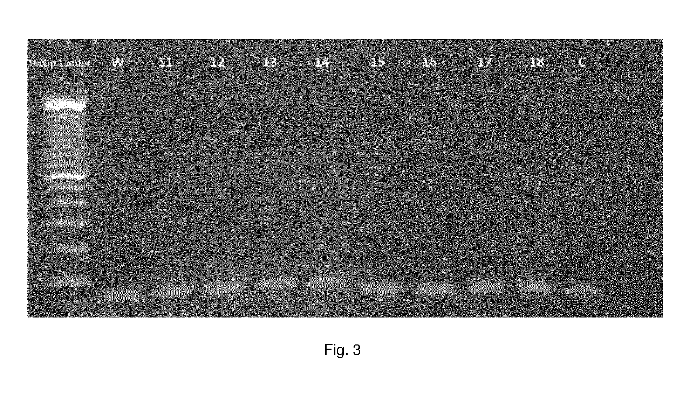

Examples for two different test solutions are shown in Fig. 3.

**Lane 1-10 Calibration curve**

| Enzyme test solution 1: | Enzyme test solution 2: |
|---|---|
| Lane 11 10µl | Lane 15 10µl |
| Lane 12 15µl | Lane 16 15µl |
| Lane 13 20µl | Lane 17 20µl |
| Lane 14 25µl | Lane 18 25µl |

Example calculation enzyme test solution 1:
In enzyme test solution 1, the last visible band is in lane 12.

The detection limit, determined by calibration with the given amount of nucleic acid is 0.018 ppm (cf. 6.3.2).
dilution factor = 50µl/15 µl = 3.3
Maximum PEI amount ≤0.018*3.3≤0.059ppm

Example calculation enzyme test solution 2:
In enzyme test solution 2, all lanes 15-18 show visible bands.
Detection limit 0.018ppm
dilution factor: 50µl/25µl = 2.0
Maximum PEI amount ≤0.018*2.0 ≤ 0.036 ppm

The specification of the enzyme solution is that PEI levels have to be ≤ 0.1ppm, Thus, enzyme test solutions 1 and 2 are within specification.

In case of test solutions 1 and 2, a series of analysis mixtures with different ratio of test solution to DNA is examined. A more precise information about the maximum PEI amount can be gained than in a case wherein only one ratio of test solution to DNA is examined, as in example 1. It cannot only be determined whether the PEI concentration is below a detection limit, but also a more precise range is gained, as shown by the different PEI ranges in test solution 1 and 2.

Selection the appropriate detection limit:
In this example, the detection limit of 0.018 was chosen in view of two boundary conditions:
   a) The specification of the enzyme solution is that PEI levels have to be ≤ 0.1ppm
   b) The dilution factor should be 5 in maximum, with a total volume of PCR volume of 50 µl

The detection limit can be set by using a suitable amount of DNA in calibration.

Assuming that in a test solution 3 a dilution factor of 5 (10 µl test solution) leads to a visible band and a dilution factor of 3.3 (15 µl test solution) does not lead to a visible band any more, the calculation is as follows:
Maximum PEI amount ≤ 0.018*5 ≤ 0.09 ppm.

It is clear that test solution 3 is still within the specification of ≤ 0.1ppm. In this example, a test solution is still within the specification, as long as one visible band exists, in the analysis mixture with the highest dilution. The case of no visible band leads to the conclusion that an enzyme test solution is out of specification, as explained below.

### 6.3.4 Alternansucrase testing - out of specification

The specification of the enzyme solution is that PEI levels have to be ≤ 0.1ppm. If the amount of PEI is higher, the sample is out of specification.

In case of no visible band, even for the lowest (10µl Alternansucrase solution) amount, the sample is out of specification and a calculation for more than x ppm (instead of less than) is possible only.

Calculation:
Take the lowest amount of enzyme test solution that has no visible band in this test (which is 10µl). Calculate the dilution factor by dividing the PCR volume to the lowest amount:
   Dilution factor = PCR volume [µl] / enzyme volume [µl] = 50 µl /10 µl

Dilution factor is 5.

Multiply the detection limit from the calibration curve to the calculated dilution factor of 5:
Minimum PEI amount > Detection limit * dilution factor > 0.018 * 5
Minimum PEI amount > 0.09

The result is the minimum amount of PEI in the enzyme test solution.

## Claims

1. Method for analysis of the presence, amount and/or concentration of a nucleic acid-binding substance in a test sample, comprising
a) formation of an analysis mixture, comprising
a test sample to by analyzed,
and nucleic acid,
wherein in the analysis mixture the nucleic acid-binding substance, if present in the test sample, binds to the nucleic acid,
b) determination of the presence, amount and/or concentration of free nucleic acid in the analysis mixture, wherein free nucleic acid is nucleic acid that is not bound to the nucleic acid-binding substance,
c) determination of the presence, amount and/or concentration of the nucleic acid-binding substance in the analysis mixture and/or in the test sample.

2. Method according to claim 1, wherein the analysis mixture comprises components for amplifying the nucleic acid by a Polymerase Chain Reaction, and wherein the analysis mixture is subjected to conditions of a Polymerase Chain Reaction.

3. Method according to one of the preceding claims, wherein analysis mixtures with different ratios of test sample to nucleic acid are formed and analyzed.

4. Method according to one of the preceding claims, further comprising the following steps:
i. formation of calibration mixtures, comprising predetermined ratios of nucleic acid-binding substance to nucleic acid, and/or predetermined amounts of nucleic acid-binding substance and nucleic acid and/or predetermined concentrations of nucleic acid-binding substance and nucleic acid,
ii. determination of the presence, amount and/or concentration of free nucleic acid in the calibration mixtures,
iii. determination of a detection limit for the nucleic acid-binding substance.

5. Method according to claim 4, wherein
- the formation of calibration mixtures comprises: formation of a series of calibration mixtures with increasing or decreasing ratio of nucleic acid-binding substance to nucleic acid,
- the determination of the detection limit comprises:
identification of a last calibration mixture in the series wherein the presence of free nucleic acid is determined, and/or of a first calibration mixture in the series wherein
no presence of free nucleic acid is determined, and/or
identification of a last calibration mixture in the series wherein no presence of free nucleic acid is determined and/or of a first calibration mixture in the series wherein
the presence of free nucleic acid is determined.

6. Method according to one of claims 4-5, wherein the calibration mixtures comprise components for amplifying the nucleic acid by a Polymerase Chain Reaction, and the calibration mixtures are subjected to conditions of a Polymerase Chain Reaction.

7. Method according to one of claims 4-6, comprising
- formation of a series of analysis mixtures with different ratios of test sample to nucleic acid,
- determination of the presence, an amount, a concentration, an amount-range or a concentration-range of nucleic acid-binding substance in the test sample, by use of the detection limit for nucleic acid-binding substance.

8. Method according to one of the preceding claims, wherein detection of free nucleic acid is carried out with electrophoresis.

9. Method according to one of the preceding claims, wherein the test sample is a cell extract.

10. Method according to one of the preceding claims, wherein the nucleic acid-binding substance is a substance that coagulates, precipitates and/or flocculates nucleic acid in/from solution.

11. Method according to one of the preceding claims, wherein the nucleic acid-binding substance is a polymer.

12. Method according to claim 11, wherein the nucleic acid-binding substance is a cationic polymer.

13. Method according to one of the preceding claims, wherein the nucleic acid-binding substance is an inorganic substance.

14. Kit, comprising nucleic acid-binding substance and nucleic acid, wherein the kit is adapted to form calibration mixtures, comprising predetermined ratios of nucleic acid-binding substance to nucleic acid, and/or predetermined amounts of nucleic acid-binding substance and nucleic acid, and/or predetermined concentrations of nucleic acid-binding substance and nucleic acid.

15. A set of calibration mixtures, comprising predetermined ratios of nucleic acid-binding substance to nucleic acid, and/or predetermined amounts of nucleic acid-binding substance and nucleic acid, and/or predetermined concentrations of nucleic acid-binding substance and nucleic acid, wherein the calibration mixtures are adapted to be analyzed on the presence, amount and/or concentration of free nucleic acid in the calibration mixtures, wherein free nucleic acid is nucleic acid that is not bound to the nucleic acid-binding substance.


SEQUENCE LISTING

<110>  aevotis GmbH

<120>  Method for analysis of a nucleic acid-binding substance in a test
        sample

<130>  P13796EP

<160>  2

<170>  PatentIn version 3.5

<210>  1
<211>  24
<212>  DNA
<213>  Artificial Sequence

<220>
<223>  primer sequence

<400>  1
gagtatgagt attcaacatt tccg                                              24


<210>  2
<211>  23
<212>  DNA
<213>  Artificial Sequence

<220>
<223>  primer sequence

<400>  2
cttggtctga cagttaccaa tgc                                               23